# EUROPEAN PATENT SPECIFICATION

(11) **EP 3 717 072 B1**
(45) Date of publication and mention of the grant of the patent: **12.10.2022**
(21) Application number: 18803678.4
(22) Date of filing: 19.11.2018
(51) Int. Cl.: A61Q 5/06, A61K 8/81

(54) **A PROCESS FOR STYLING HUMAN HAIR**
VERFAHREN ZUM STYLING VON MENSCHLICHEN HAAREN
PROCÉDÉ PERMETTANT DE COIFFER LES CHEVEUX HUMAINS

(30) Priority: 29.11.2017 EP 17204291
(43) Date of publication of application: 07.10.2020
(73) Proprietor: BASF SE, 67056 Ludwigshafen am Rhein (DE)
(72) Inventor: FALKOWSKI, Juergen, 40589 Düsseldorf-Holthausen (DE); TOMLINSON, Andrea, SK8 6QG Cheadle (GB); WIETHOFF, Helena, 40589 Düsseldorf-Holthausen (DE); KURTH, Nicole, 40789 Monheim (DE)
(74) Representative: BASF IP Association
(86) International application number: PCT/EP2018/081677
(87) International publication number: WO 2019/105777

(56) References cited:
- WO-A1-2005/087186
- US-A- 5 059 414
- US-A1- 2003 143 180
- US-A1- 2015 118 175
- DATABASE GNPD [Online] MINTEL; June 2007 (2007-06), "Super Hold Styling Sculptor", XP002780718, Database accession no. 719281
- DATABASE GNPD [Online] MINTEL; October 2009 (2009-10), "Frizz Control and Straightener Serum", XP002780719, Database accession no. 1187114
- DATABASE GNPD [Online] MINTEL; May 2016 (2016-05), "Define Style Smoothing Fluid", XP002780720, Database accession no. 3994761
- DATABASE GNPD [Online] MINTEL; January 2013 (2013-01), "Hair Styling Balm", XP002780721, Database accession no. 1976840
- DATABASE GNPD [Online] MINTEL; October 2016 (2016-10), "Grid Structural Setting Spray", XP002780722, Database accession no. 4304859
- DATABASE GNPD [Online] MINTEL; November 2015 (2015-11), "Heat Tamer", XP002780723, Database accession no. 3558967
- DATABASE GNPD [Online] MINTEL; June 2012 (2012-06), "Heat Defence Spray", XP002780724, Database accession no. 1806263
- DATABASE GNPD [Online] MINTEL; February 2008 (2008-02), "Aerostraight Spray-In Straightener", XP002780725, Database accession no. 863554
- B.SCHWEID, G.MARTINO: "How Thermal Protection is Made Possible with Sodium Polystyrene Sulfonate", SOFW Journal, vol. 130, no. 8 2004, pages 36-40, XP002780972, Retrieved from the Internet: URL:http://www.sofw.com/index/sofw_en/Sear ch.html?cosearch=schweid+martino&coshow=na +308&costart=1
- Anonymous: "Product Data Sheet RHEOCARE HSP-1180 formerly COSMEDIA HSP-1180", , 8 February 2019 (2019-02-08), XP055553937, Retrieved from the Internet: URL:https://biakhim.ua/index.php?option=co m_k2&itemid1173&id.. [retrieved on 2019-02-08]

## Description

The present invention relates to a composition comprising a polymer, wherein this polymer is water-soluble and has a melting point of at least 145° C and comprises sulfonic acid groups, and an organic amine having at least two amino groups, and optionally further cosmetic ingredients. Furthermore, the present invention relates to a process for restyling human hair comprising applying to the human hair a composition comprising a polymer, wherein this polymer is water-soluble and has a melting point of at least 145° C and comprises sulfonic acid groups, and an organic amine having at least two amino groups, and optionally further cosmetic ingredients, thereafter heat-treating the hair, together with the composition on its surface, thereafter allowing the hair to cool down to a temperature of below 100° C, and thereafter changing the hair style by heat-treating the hair. Furthermore, the present invention relates to the use of the said composition for restyling human hair.

Hair Styling products containing water or ethanol soluble polymers are very popular products in today's cosmetic market. Such products typically involve a liquid or wax/gel-like composition which is applied on wet or dry hair to obtain a certain long-lasting hair style. They can be produced as aerosol sprays, sprays, mousses, gels, waxes, serums or other textures. Apart from the polymer respectively the polymer combination a large number of ancillary ingredients is used to adjust certain texture or performance effects.

The most important performance of hair styling products is the stiffness respectively hold of the hair style and the ability to resist high humidity conditions. Natural hair can also be styled by using blow dryers or straightening/curling devices. The most important disadvantages of only heat-treated hair are the low longevity of the hair style and the sensitivity against moisture. Similar to synthetic polymers natural hair also has a glass transition temperature (T_{g}) depending on its water content. The T_{g} of absolute dry human hair (0% water content) is around 144°C. It decreases with increasing water content and reaches about 0° C at about 23 % water content.

The humidity condition to obtain very low water content (< 5 wt.-%) of hair can almost be achieved during hair straightening with heated devices. Straightening irons therefore should be operated at least at 150 °C for styling hair. The relative humidity within buildings is typically between 40 % RH and 60 % RH. Depending on season and weather conditions the relative humidity outside can be between 20 % RH and 90 % RH. As a consequence, natural hair takes up water and the T_{g} decreases below room temperature or ambient temperature. If the hair has only been blow-dried or heat-treated the hair style is lost and a new hair styling procedure is necessary. Hair styling products containing humidity resistant polymers avoid this drawback of natural hair.

Devices like straightening irons nevertheless are very popular because they can - depending on consumer preference - either straighten curly hair or curl straight hair. As mentioned before a temperature for these devices of at least 150° C is necessary to form hair to another style. Many straightening irons are using even temperatures of up to 230° C to boost or shorten the styling procedure. The major drawback of these high-temperature treatments is the irreversible damage of the hair at temperatures beyond about 180° C and especially beyond about 200°C. There are some products in the hair styling market claiming heat-protection in this temperature range. These products are forming films around the hair fibers and try to prevent a direct contact of the heated the iron with outer hair surface called cuticle. Nevertheless - depending on consumer habits - they cannot prevent hair damages completely. Many straightening irons or crimpers are therefore nowadays operating at lower temperatures, like 170° C - 185°C to prevent hair damage.

Most synthetic polymers have a glass transition temperature T_{g}. There are standard polymers like PP which are continuously produced using special catalysts to adjust molecular weight distribution and thus the melting point and T_{g}. These polymers can be melted easily due to their narrow molecular weight distribution and they are for example used in the production of synthetic fibers/fabrics in a melt process. Polymers which are typically used in the cosmetic industry should in general be water soluble or ethanol soluble. They are typically produced in batch processes and most of them comprise special monomers to adjust parameters like solubility, water resistance or sensorial properties. They also have measurable glass transition temperatures but their melting behavior is different from polymers like PP and normally there is no real liquid/low viscous phase transition and some of them even decompose at higher temperatures. If these polymers are applied on hair and treated at high temperatures like 200°C they are often sticky and highly viscous. These physical properties have a negative impact on the application. The sliding or gliding of straightening irons is hampered and in some cases the residence time of the heated iron on hair is increased resulting in further hair damage.

It is desirable that hair styling products allow easy restyling of hair. Restyling means that after a first hair style has been made, and has been worn for some time, the hair style is changed, e. g. from curly to flat or vice versa.

US2003143180 A1 discloses a process for restyling the hair comprising applying a composition comprising a film-forming fixing polymer and a crystalline hot-melt polymer to the hair, styling by heating the hair to above the crystalline melting temperature of the crystalline hot-melt polymer, and cooling, whereby the steps of heating and cooling may be repeated many times without the application of additional styling composition. The polymer has a crystalline melting temperature of 30 to 80°C. The film-forming fixing polymers may be sulphonic acid polymers which may be neutralised.

The problem underlying the present invention is to provide a process for restyling human hair which results in low or no damage to the hair, i. e. which operates at moderate temperatures, and which results in a styling effect that is more stable than simply heat treated natural hair. Furthermore, the problem underlying the present invention is to provide compositions for restyling human hair.

This problem is solved by the composition according to the present invention and by the process according to the present invention. This composition is one subject of the present invention. This process is another subject of the present invention.

The composition according to the present invention A composition comprising a polymer, wherein this polymer is water-soluble and has a melting point of at least 145° C, preferably at least 160° C, and comprises sulfonic acid groups, and a specific organic amine having at least two amino groups, and optionally further cosmetic ingredients, preferably selected from the group consisting of solvents, rheology modifiers, preservatives, surfactants, emulsifiers, perfumes, pigments and coloring agents.

The process according to the present invention is a process for restyling human hair comprising applying to the human hair a composition comprising a polymer as defined in the previous paragraph and an organic amine, selected from the group consisting of ethylendiamine, diethylenetriamine, triethylendiamine, tetraethylenepentamine and tetrahydroxypropyl ethylenediamine, preferably in an amount as defined in claim 5, and optionally further cosmetic ingredients, thereafter heat-treating the hair, together with the composition on its surface, at a temperature of 145 to 250° C, preferably 150 to 230° C, more preferably 150 to 190°C, more preferably 160 to 185° C, wherein the hair-style is changed during this heat treatment, thereafter allowing the hair to cool down to a temperature of below 100° C, and thereafter changing the hair style by heat-treating the hair, together with the composition on its surface, at a temperature of 145 to 250° C, preferably 150 to 230° C, more preferably 150 to 190°C, more preferably 160 to 185° C.

Water-soluble means that at 20° C it is possible to make a solution of at least 0.1 % polymer in water, preferably at least 2 % polymer in water (without adding neutralizing agents or other additives).

The melting point of the polymer can be determined visually. The polymer in powder form is filled in a thin glass tube and heated until the initially non-transparent powder becomes clear, i. e. becomes a melt. The heating rate is chosen low so that further lowering of the heating rate does not result in determining a lower melting point.

Tetrahydroxypropyl ethylenediamine can be commercially obtained from BASF (the trademark of BASF is Neutrol^{®} TE from BASF)

In one embodiment of the composition according to the present invention or of the process according to the present invention a formulation is used and the formulation comprises at least one further polymer, wherein this further polymer is used preferably at a concentration of 0.1 to 10% by weight.

In one embodiment of the composition according to the present invention or of the process according to the present invention a formulation is used and the formulation comprises at least one further polymer, wherein this further polymer is cationic and used preferably at a concentration of 0.1 to 10% by weight.

In one embodiment of the composition according to the present invention or of the process according to the present invention a formulation is used and the formulation comprises at least one further polymer, wherein this further polymer is anionic and used preferably at a concentration of 0.1 to 10% by weight.

In one embodiment of the composition according to the present invention or of the process according to the present invention a formulation is used and the formulation comprises at least one further polymer, wherein this further polymer is nonionic and used preferably at a concentration of 0.1 to 10% by weight.

In one embodiment of the composition according to the present invention or of the process according to the present invention a formulation is used and the formulation comprises a combination of different polymers, wherein these further polymers are used together at a total concentration of 0.1 to 15% by weight, preferably at a total concentration of 0.1 to 10% by weight.

The combination with a cationic polymer in general leads to a higher stiffness and to a better hold of the hair style, curl retention and weather resistance are improved by the presence of the cationic polymer.

In one embodiment of the present invention the polymer in the composition according to the present invention is selected from the group consisting of polyacrylamidomethylpropane sulfonic acid and polystyrene sulfonic acid, preferably the polymer is polyacrylamidomethylpropane sulfonic acid.

The described organic neutralizing agents can also be combined so that a mixture of inorganic and organic neutralizing agents is used or using a mixture of different organic neutralizing agents is used in an amount that leads to partial or complete neutralization of the polymer, so that the composition preferably has a pH value of 1 to 14, preferably 3 to 9.

Surprisingly it has been found that the melting behavior and the styling effect after heat activation of the preferred polymer can be adjusted by the choice of the neutralizing agent. Example 6 describes this surprising effect by measuring bending stiffness values of heat treated hair strands. The use of different neutralizing agents results in significantly different stiffness values after heat treatment. Organic neutralizing agents yield to high stiffness values presumably because the heat transfer from the straightening iron can probably melt the polymer on the hair strand and thus reconnect the hair fibers after cooling. This surprising process does not work in formulations containing polyacrylamidomethylpropane sulfonic acid neutralized with sodium hydroxide, but is most effective (highest stiffness value) with the organic neutralizing agent Neutrol^{®} TE from BASF (tetrahydroxypropyl ethylenediamine). There is no exact scientific explanation for the different physicochemical phase behavior of the differently neutralized polymers but it can be favorably used to tweak formulations with respect to stiffness or suppleness. It is assumed that the presence of more than one amio group in the amine used to neutralize the polymer contributes to the superior properties of the compositions comprising tetrahydroxypropyl ethylenediamine.

Example 7 shows that the bending stiffness values of only dried polymer solutions are all on the same level and practically independent from the neutralizing agent. The absolute bending stiffness values are higher than in Example 6 because the dried strands have a larger cross-sectional area when they were broken by the cantilever of the test instrument.

Hair strands treated with differently neutralized agents of the preferred polymer can also be used to restyle the hair with different styling devices, like hair straighteners, crimp irons or curling irons in the preferred temperature range from 150 °C to 190 °C. Example 8, 9 and 10 show this surprising effect. In compliance with the bending stiffness values from example 6 best restyling effects can be obtained when Neutrol^{®} TE is used as a neutralizing agent. Other organic amines, like triethanolamine or aminomethyl propanol also lead to good results. Examples 11 and 12 compare this surprising effect with formulations containing different neutralizing agents. It can be clearly seen on the images (figures) that the hair strands treated with the inorganic neutralizing agent have spread, best styling results can be achieved with Neutrol^{®} TE. The restyling procedure can also be repeated with different styling devices because the melting of the polymer is a reversible process. The hair itself is not damaged during these restyling procedures because of the moderate temperature range from 150 °C to 190°C and the choice of the neutralizing agent allows the adjustment of the stiffness of the hair style, like curly or straight hair.

As described above the choice of the neutralizing agent influences the stiffness of the heat-treated hair strands. On the images (figures) of Examples 11 and 12 it could also be shown that there is less frizz on hair strands treated and heat activated with the preferred neutralizing agents and the polymer polyacrylamidomethylpropane sulfonic acid. Especially hair strands showing higher stiffness values are almost free from frizz after heat activation in the preferred temperature range from 150 °C to 190 °C. This is also a main advantage compared to only water-treated strands which are styled in this temperature range.

Example 13 shows that the anti-frizz effect of a formulation containing Neutrol^{®} TE works also when the hair strand had been exposed to high humidity.

In one embodiment of the present invention the composition is a rinse-off composition selected from the group consisting of a hair shampoo and a shower gel, each containing anionic surfactants or betaine surfactants or nonionic surfactants.

In one embodiment of the present invention the composition is a conditioner containing at least one anionic, nonionic or cationic surfactant.

Another subject of the present invention is the use of a composition as defined before for styling human hair. Preferably this use is carried out in a process according to the present invention.

In one embodiment of the process according to the present invention the formulation used in this process is selected from the group consisting of a gel, a mousse, a spray, a serum, a cream and a wax.

In one embodiment of the composition according to the present invention the composition is a non-aerosol composition, and the composition is selected from the group consisting of a gel, a mousse, a spray, a serum, a cream and a wax.

The polymer according to the present invention is water-soluble and thus can be easily washed out of the hair.

The polymer according to the present invention can be "heat-activated" by melting it. This polymer can be combined with other cationic, anionic or nonionic polymers, preferably in non-aerosol formulations.

The polymer according to the present invention can be used in formulations, preferably non-aerosol formulations, containing at least one further, cationic polymer, e. g. Polyquaternium-4, Polyquaternium-11, Polyquaternium-16, Polyquaternium-46 or Polyquaternium-68.

The polymer according to the present invention can be used in formulations, preferably non-aerosol formulations, containing at least one further, anionic polymer, e. g. Acrylates Copolymers, Acrylate/Methacrylamide Copolymers, Acrylates/t-Butylacrylamide Copolymers, Vinylacetat/Crotonates Copolymers, Vinylacetat/Crotonates/Vinyl Neodecanoate Copolymers or Polyurethanes. In can also be used in formulations further comprising at least one further nonionic polymer, e. g. polyvinylpyrrolidon, Polyvinylpyrrolidon/Vinylacetate Copolymers, Polyvinylcaprolactams or Polyvinylpyrrolidon/Methacrylamide/Vinylimidazole Copolymers.

The formulations or compositions according to the present invention can comprise solvents, e. g. ethanol or propanol. They can also comprise other ancillary ingredients like glycerin, sorbitol, panthenol, proteins, rheology modifiers, preservatives, surfactants, emulsifiers, perfumes, pigments and/or coloring agents.

The polymer according to the invention can be formulated in various hair care and styling formulations. Due to its anionic nature it can also be formulated in rinse-off formulations, e. g. in hair shampoos containing ether sulfate surfactants, like laureth sulfate, and/or betaine surfactants, like cocoamidopropyl betaine, or nonionic surfactants, like alkyl polyglucosides or ethoxylated fatty alcohols. The use in leave-on conditioners for hair care containing cationic surfactants is possible and can further enhance wet and dry combing.

A possible application procedure of the polymer according to the present invention is as follows. After the product ( formulation or composition comprising the polymer) has been applied on wet hair using e.g. a spray, hair cream, serum or conditioner, the hair is dried by using a blow-dryer and a combing device, like brushes or combs. The polymer facilitates the blow-drying/combing procedure and the polymer is homogenously distributed on the hair surface. Afterwards the applied polymer can be "heat-activated" using commercially available styling devices like straightening irons, curling irons or crimpers.

Due to the lubricity of the polymer according to the present invention the application of the styling devices is smooth-running compared to other polymers which do not change their phase in the preferred temperature range from 150 °C to 190 °C. The sliding or gliding of the styling devices is not hampered and the residence time of the heated iron on hair is further reduced. That is why possible damages of the hair are further diminished.

If desired it is also possible to restyle the hair, like curling of straightened hair, whilst the polymer film is on the hair. The polymer film can also be easily washed out using standard hair shampoos.

All in all the composition according to the present invention offers a lot of new features for hair styling and hair care formulations to prevent hair damages due to styling or restyling with heat treatment devices.

The polymer according to the present invention can be applied to surfaces like human hair or natural respectively artificial fibers. They are especially useful for hair styling products like gels, creams, mousses serums or sprays to prevent hair damage during procedures like hair straightening or hair curling at elevated temperatures.

Further cosmetic ingredients suitable for use in the compositions according to the present invention have been described in the previous paragraphs. Furthermore, the ingredients used in the examples of the present text are suitable. Furthermore, there are numerous textbooks known to the person skilled in the art listing cosmetic ingredients suitable for use in the compositions according to the present, e. g. the "International Cosmetic Ingredient Dictionary and Handbook", 16^{th} edition, 2016, edited by the CTFA.

### Examples

Concentrations are given in weight-% (wt%) unless another unit is given explicitly.

Concentrations mean concentrations of active matter (AM), unless another definition is given explicitly.

Rheocare^{®} HSP 1180 from BASF which is used for the following examples and formulations is a product that has a polymer content of between 14 wt.-% AM and 18 wt.-% AM (active matter). Thus, the use of 12.5 wt.-% Rheocare^{®} HSP 1180 in a formulation means 2 wt.-% AM polymer in the resulting formulation.

### Example 1

Following polymer solutions were prepared:
- Polyacrylamidomethylpropane Sulfonic Acid (Rheocare^{®} HSP 1180 from BASF), 1 wt.-% active matter, adjusted to pH = 6 by aminomethyl propanol
- Polyacrylamidomethylpropane Sulfonic Acid (Rheocare^{®} HSP 1180 from BASF), 2 wt.-% active matter, adjusted to pH = 6 by aminomethyl propanol
- Polyacrylamidomethylpropane Sulfonic Acid (Rheocare^{®} HSP 1180 from BASF), 2 wt.-% active matter, adjusted to pH = 4 by aminomethyl propanol
- Polyacrylamidomethylpropane Sulfonic Acid (Rheocare^{®} HSP 1180 from BASF), 5 wt.-% active matter, adjusted to pH = 6 by aminomethyl propanol

In each experiment one hair strand (Caucasian hair, 2 g/ 15 cm length, round-shaped) was treated with these different polymer solutions by dipping twice into the solution. The redundant product was removed by squeezing with fingers. Then the wet hair strand was dried and combed-out. Then bending stiffness after dry combing and after heat treatment using a straightening iron (supplier: Remington; Sleek and Curl, S1051) at 150° C respectively 160° C was determined using a 3-point method (this was done with a T.A.Plus Texture Analyzer; supplier: Stable Micro Systems) in a climatized chamber at 21° C and 65 % relative humidity. The following values were obtained:

| | Bending Stiffness after dry combing | Bending Stiffness after heat treatment at 150 °C | Bending Stiffness after heat treatment at 160 °C |
|---|---|---|---|
| 1 wt.-%, pH = 6 | 14 cN | 16 cN | 22 cN |
| 2 wt.-%, pH = 6 | 13 cN | 30 cN | 56 cN |
| 2 wt.-%, pH = 4 | 13 cN | 27 cN | 87 cN |
| 5 wt.-%, pH = 6 | 20 cN | 88 cN | 88 cN |

The higher the value of the bending stiffness the more effective and stable is the styling of the hair.

### Example 2

The following polymer solutions diluted to 2 wt.-% active matter and adjusted to pH = 6 were tested:
- Polyacrylamidomethylpropane Sulfonic Acid (Rheocare^{®} HSP 1180 from BASF)
- Acrylates Copolymer (Luviflex^{®} Soft from BASF)
- VP/Methacrylamide/Vinyl Imidazole Copolymer (Luviset^{®} Clear from BASF)

In each case 10 hair strands (Caucasian hair, 2 g/ 15 cm length, round-shaped) were treated with these different polymer solutions by dipping twice into the solution. The redundant product was removed by squeezing with fingers. Then the wet hair strands were dried by hanging vertically in a climatized chamber at 21° C and 65 % relative humidity (RH). Finally the bending stiffness after drying, after dry combing and after heat treatment using a straightening iron at 160° C was determined using a 3-point method (T.A.Plus Texture Analyzer; supplier: Stable Micro Systems). The following values were obtained:

| | Bending Stiffness after drying | Bending Stiffness after dry combing | Bending Stiffness after heat treatment |
|---|---|---|---|
| Rheocare^{®} HSP 1180 | 573 +/- 76 cN | 34 +/- 12 cN | 63 +/- 2 cN |
| Luviflex Soft | 390 +/- 40 cN | 29 +/-5 cN | 25 +/- 5 cN |
| Luviset Clear | 674 +/- 90 cN | 28 +/- 3 cN | 32 +/- 8 cN |

Only Rheocare^{®} HSP 1180 resulted in a significant increase of bending stiffness after heat treatment.

### Example 3

10 hair strands (Caucasian hair, 2 g / 15 cm length, round-shaped) were dipped twice into a 2 wt.-% solution of polyacrylamidomethylpropane sulfonic acid which had been adjusted to pH = 6 by aminomethyl propanol. The redundant product was removed by squeezing with fingers. Then the wet hair strands were blow-dried using a commercially available blow dryer and simultaneously combed during the blow drying procedure. Finally the bending stiffness after blow drying/combing and after heat treatment using a straightening iron at 160° C was determined in a climatized chamber at 21° C and /65 % RH using a 3-point method (T.A.Plus Texture Analyzer; supplier: Stable Micro Systems). The following values were obtained:

| | After Blow Drying/Combing | After Heat Treatment |
|---|---|---|
| Bending Stiffness | 32 +/- 6 cN | 259 +/- 52 cN |

It can be concluded that combing during blow drying improves the distribution of the polymer and thus results in a higher increase of the bending stiffness after heat treatment.

### Example 4

10 hair strands (Caucasian hair, 2 g/ 15 cm length, round-shaped) were dipped twice into a 2 wt.-% solution of polyacrylamidomethylpropane sulfonic acid which had been adjusted to pH = 1.5 by aminomethyl propanol. The redundant product was removed by squeezing with fingers. Then the wet hair strands were blow-dried using a commercially available blow dryer and simultaneously combed during the blow drying procedure. Finally the bending stiffness after blow drying/combing and after heat treatment using a straightening iron at 160° C was determined in a climatized chamber at 21° C and 65 % RH using a 3-point method (T.A.Plus Texture Analyzer ;supplier: Stable Micro Systems). The following values were obtained:

| | After Blow Drying/Combing | After Heat Treatment |
|---|---|---|
| Bending Stiffness | 35 +/- 8 cN | 216 +/- 34 cN |

The comparison of examples 3 and 4 shows that pH 1.5 and 6 lead to comparable results.

### Example 5

The following freeze-dried samples of polymer solutions were compared according to ISO 6321 (e.
- polyacrylamidomethylpropane Sulfonic Acid (Rheocare^{®} HSP 1180 from BASF), adjusted to pH = 6 by aminomethyl propanol
- vinylpyrrolidon/vinylacetate-copolymer (Luviskol^{®} VA 64 from BASF)
It was found that polyacrylamidomethylpropane Sulfonic Acid (Rheocare^{®} HSP 1180 from BASF) has a melting point at about 160° C, whereas vinylpyrrolidon/vinylacetate-copolymer (Luviskol^{®} VA 64 from BASF) decomposes at about 260° C without melting.

### Example 6

Following formulations with different neutralizing agents (composition see example formulations) were prepared:
- Formulation no. HB-DE-16-121-001 (neutralized with sodium hydroxide)
- Formulation no. HB-DE-16-121-008 (neutralized with sodium hydroxide)
- Formulation no. HB-DE-16-121-012 (neutralized with aminomethyl propanol)
- Formulation no. HB-DE-16-121-013 (neutralized with Neutrol^{®} TE)
- Formulation no. HB-DE-16-121-018 (neutralized with aminomethyl propanol)
- Formulation no. HB-DE-16-121-019 (neutralized with sodium hydroxide)
- Formulation no. HB-DE-16-121-020 (neutralized with aminomethyl propanol)
- Formulation no. HB-DE-16-121-021 (neutralized with triethanolamine)

In each experiment 10 hair strands (Caucasian hair, 2 g/ 15 cm length, round-shaped) were treated with these different formulations by dipping twice into the solution. The redundant product was removed by squeezing with fingers. Then the wet hair strands were dried and combed-out. After heat treatment using a straightening iron at 160° C the bending stiffness of the flattened hair strands were determined using a 3-point method (this was done with a T.A.Plus Texture Analyzer; supplier: Stable Micro Systems) in a climatized chamber at 21° C and 65 % relative humidity. The following values were obtained:

| **Formulation no.** | **Bending Stiffness after heat treatment at 160 °C [ cN ]** |
|---|---|
| only water | 24.1 +/- 6.8 |
| HB-DE-16-121-001 | 16.1 +/- 1.5 |
| HB-DE-16-121-008 | 17.3 +/- 1.3 |
| HB-DE-16-121-012 | 106.2 +/- 13.3 |
| HB-DE-16-121-013 | 192.4 +/- 32.1 |
| HB-DE-16-121-018 | 21.9 +/- 3.9 |
| HB-DE-16-121-019 | 64.1 +/- 27.2 |
| HB-DE-16-121-020 | 101.4 +/- 7.3 |
| HB-DE-16-121-021 | 97.09 +/- 8.9 |

The formulations HB-DE-16-121-001, HB-DE-16-121-013, HB-DE-16-121-020 and HB-DE-16-121-021 had the same chassis, only the neutralizing agents had been changed:

| **Formulation no.** | **Neutralizing Agent** | **Bending Stiffness after heat treatment at 160 °C [ cN ]** |
|---|---|---|
| HB-DE-16-121-001 | Sodium Hydroxide | 16.1 +/- 1.5 |
| HB-DE-16-121-013 | Neutrol TE | 192.4 +/- 32.1 |
| HB-DE-16-121-020 | Aminomethyl propanol | 101.4 +/- 7.3 |
| HB-DE-16-121-021 | Triethanolamine | 97.09 +/- 8.9 |

### Example 7

Following formulations with same chassis, but different neutralizing agents (composition see example formulations) were prepared:
- Formulation no. HB-DE-16-121-001 (neutralized with sodium hydroxide)
- Formulation no. HB-DE-16-121-013 (neutralized with Neutrol^{®} TE)
- Formulation no. HB-DE-16-121-020 (neutralized with aminomethyl propanol)
- Formulation no. HB-DE-16-121-021 (neutralized with triethanolamine)

In each experiment 10 hair strands (Caucasian hair, 2 g/ 15 cm length, round-shaped) were treated with these different formulations by dipping twice into the solution. The redundant product was removed by squeezing with fingers. Then the wet hair strands were only dried overnight in a climatized chamberer at 21°C and 65% relative humidity. Then bending stiffness of the round-shaped and dry strands were determined using a 3-point method (this was done with a T.A.Plus Texture Analyzer; supplier: Stable Micro Systems) in a climatized chamber at 21° C and 65 % relative humidity. The following values were obtained:

| **Formulation no.** | **Neutralizing Agent** | **Bending Stiffness after Drying at 21 °C [ cN ]** |
|---|---|---|
| HB-DE-16-121-001 | Sodium Hydroxide | 465.5 +/- 48.8 |
| HB-DE-16-121-013 | Neutrol TE | 476.2 +/- 24.8 |
| HB-DE-16-121-020 | Aminomethyl propanol | 484.7 +/- 44.7 |
| HB-DE-16-121-021 | Triethanolamine | 399.4 +/- 42.1 |

### Example 8

One hair strand treated with formulation no. HB-DE-16-121-013 from example 6 which had been activated by a straightening iron at 160 °C was restyled using a Babyliss Pro from Miracurl to get a curly strand (figure 1, left). Afterwards the same strand was treated again using a straightening iron (Braun Satin Hair 7) to get straight hair (figure2, middle). Finally, the strand was treated using a curling iron (BaByliss Pro fashion attitude) to get definitely curled hair (figure 3, right).

The procedures can be continued with other styling devices adjusted to the preferred temperature range from 160 °C - 185 °C without damaging the hair. If desired the product can be washed out.

It is also possible to spray e.g. formulation no. HB-DE-16-121-013 onto single hair strands on the head to create special hair style effects with better hold and humidity resistance than only water-treated strands (see example 8).

### Example 9

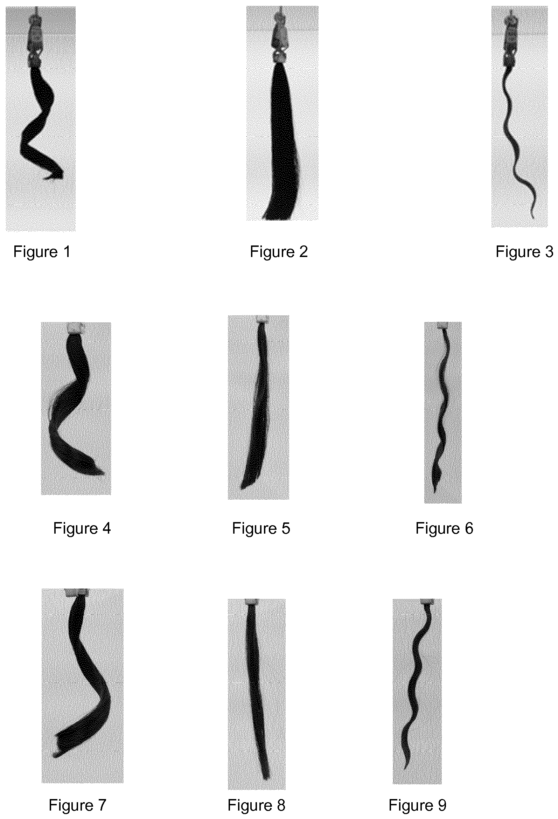

One hair strand treated with formulation no. HB-DE-16-121-020 from example 6 which had been activated by a straightening iron at 160 °C were restyled using a Babyliss Pro from Miracurl to get a curly strand (figure 4, left).

Afterwards the same strands were treated again using a straightening iron (Braun Satin Hair 7) to get straight hair (figure 5, middle). Finally, the strand was treated using a curling iron (BaByliss Pro fashion attitude) to get definitely curled hair (figure 6, right).

### Example 10

One hair strand treated with formulation no. HB-DE-16-121-021 from example 6 which had been activated by a straightening iron at 160°C were restyled using a Babyliss Pro from Miracurl to get a curly strand (figure 7, left).

Afterwards the same strands were treated again using a straightening iron (Braun Satin Hair 7) to get straight hair (figure 8, middle). Finally, the strand was treated using a curling iron (BaByliss Pro fashion attitude) to get definitely curled hair (figure 9, right).

### Example 11

Four hair strands treated with formulation no. HB-DE-16-121-001, HB-DE-16-121-013, HB-DE-16-121-020 and HB-DE-16-121-021 (same chassis, but different neutralizing agent) from example 6 which had been activated by a straightening iron at 160 °C were compared after restyling using a using a curling iron (BaByliss Pro fashion attitude).

| **Formulation no.** | **Neutralizing Agent** | **Figure no.** |
|---|---|---|
| HB-DE-16-121-001 | Sodium Hydroxide | 10 |
| HB-DE-16-121-013 | Neutrol TE | 11 |
| HB-DE-16-121-020 | Aminomethyl propanol | 12 |
| HB-DE-16-121-021 | Triethanolamine | 13 |

### Example 12

Four hair strands treated with formulation no. HB-DE-16-121-001, HB-DE-16-121-013, HB-DE-16-121-020 and HB-DE-16-121-021 (same chassis, but different neutralizing agent) from example 6 which had been activated by a straightening iron at 160 °C were compared after restyling using a using a crimp iron (Ghd contour professional). Figures (14, 15, 16, 17) taken from the side of the hair strands to visualize the styling effect.

| **Formulation no.** | **Neutralizing Agent** | **Figure no.** |
|---|---|---|
| HB-DE-16-121-001 | Sodium Hydroxide | 14 |
| HB-DE-16-121-013 | Neutrol TE | 15 |
| HB-DE-16-121-020 | Aminomethyl propanol | 16 |
| HB-DE-16-121-021 | Triethanolamine | 17 |

### Example 13

The hair strand from example 8 (figure 3, right, formulation no. HB-DE-16-121-013) and an only water-treated strand which has also been treated with the curling iron (BaByliss Pro fashion attitude) were put into a climate container for 18 h at 21°C / 65 % RH.

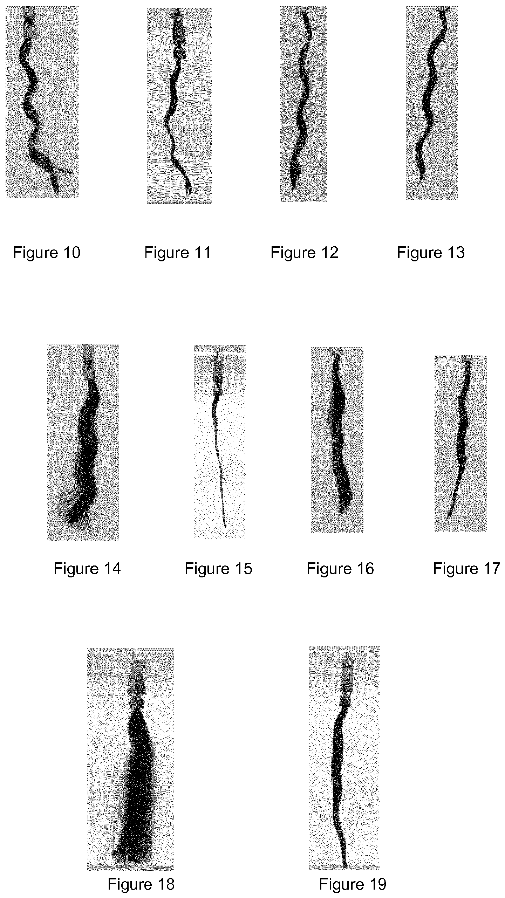

It can be seen on figure 18 that the water-treated strand has spread, the strand treated with formulation no. HB-DE-16-121-013 (figure 19) has not spread and shows no frizz.

### Interpretation of the examples

The examples show that Polyacrylamidomethylpropane Sulfonic Acid can be "heat-activated" (by melting). Hairs treated with this polymer can be easily styled at moderate temperatures using commercially available styling devices like straightening irons, curling irons or crimpers. The styling effect strongly depends on the neutralizing agents used in the formulation. The examples 6, 11 and 12 are showing this surprising effect. The use of organic neutralizing agents, especially Neutrol TE (tetrahydroxypropyl ethylenediamine), enables the restyling of hair strands and results in defined hair styles.

These devices can be run at temperatures from about 160° C to 230° C. Due to the unique performance of the polymer the preferred application temperature can be from 160°C to 185°C. Damaging the hair can be avoided in this temperature range and the applied and heat-activated polymerfilm forms the desired hair styling which is more resistant against mechanical or humidity impacts than heat-treated natural hair.

Example 3 and 4 represent the preferred application procedure. After the product has been applied on wet hair using e.g. a spray, hair cream or conditioner, the hair is dried by using a blow-dryer and a combing device, like brushes or combs. The polymer facilitates the blow-drying/combing procedure and the polymer is homogenously distributed on the hair surface. Afterwards the applied polymer can be "heat-activated" using commercially available styling devices like straightening irons, curling irons or crimpers. If desired it is also possible to restyle the hair, like curling of straightened hair, whilst the polymer film is on the hair. The polymer film can also be easily washed out using standard hair shampoos.

The following formulations have been made and tested. % means % by weight. Rheocare^{®} HSP 1180 from BASF which is used for the following formulations is a product that has a polymer content of between 14 wt.-% AM and 18 wt.-% AM. Thus, the use of 12.5 wt.-% Rheocare^{®} HSP 1180 in a formulation means 2 wt.-% AM polymer in the resulting formulation.

### Example Formulation:

**Formulation no. HB-DE-16-121-013: a pumpspray**

| | Ingredient | INCl | % |
|---|---|---|---|
| A | Aqua, demin. | Aqua | 74.40 |
| | Ethanol abs. | Alcohol | 10 |
| | Rheocare^{®} HSP 1180 | Polyacrylamidomethylpropane sulfonic acid | 12.5 |
| | Neutrol^{®} TE | Tetrahydroxypropyl ethylenediamine | qs |
| B | Gluadin^{®} Kera-P LM | Hydrolyzed vegetable protein | 2 |
| | D-Panthenol 75W | Panthenol | 0.50 |
| C | Perfume "Cotton Touch" | Perfume | 0.30 |
| | Eumulgin^{®} CO 40 | PEG-40 hydrogenated castor oil | 0.30 |
| D | Citric acid, 50% sol. | Citric acid | qs |

### Properties:

| | | |
|---|---|---|
| pH value | 4.85 | |
| Viscosity (23°C, Brookfield RVT, Sp4, 10rpm) | | 860 mPa*s |
| Appearance | slightly yellowish, clear | |
| Manufacturing process: | | |

Dissolve Rheocare HSP^{®} 1180 in a mixture of water and ethanol. Adjust pH to ~6 with Neutrol^{®} TE and add ingredients of phase B. Solubilize phase C separately and add to the combined phase A + B. If necessary adjust pH with phase D.

## Claims

1. A composition comprising
a polymer, wherein this polymer is water-soluble and has a melting point of at least 145° C, preferably at least 160° C, and comprises sulfonic acid groups,
and an organic amine having at least two amino groups, wherein the organic amine having at least two amino groups is selected from the group consisting of ethylendiamine, diethylenetriamine, triethylendiamine, tetraethylenepentamine and tetrahydroxypropyl ethylenediamine.
and optionally further cosmetic ingredients, preferably selected from the group consisting of solvents, rheology modifiers, preservatives, surfactants, emulsifiers, perfumes, pigments and coloring agents.

2. The composition according to claim 1, wherein the polymer is selected from the group consisting of polyacrylamidomethylpropane sulfonic acid and polystyrene sulfonic acid, wherein preferably the polymer is polyacrylamidomethylpropane sulfonic acid.

3. The composition according to claims 1 or 2, wherein the organic amine having at least two amino groups is tetrahydroxypropyl ethylenediamine.

4. The composition according to any of claims 1 to 3, wherein the polymer is present in an amount of from 0.1 to 10 % by weight, preferably 0.5 to 2.5 % by weight.

5. The composition according to any of claims 1 to 4, wherein the organic amine having at least two amino groups is present in an amount that leads to partial or complete neutralization of the polymer, and wherein the composition has a pH value of 1 to 14, preferably of 3 to 9.

6. The composition according to any of claims 1 to 5, wherein the composition comprises at least one further polymer, wherein this further polymer is preferably present at a concentration of 0.1 to 10 % by weight and is selected from the group consisting of nonionic, anionic or cationic polymers.

7. The composition according to any of claims 1 to 6, wherein the composition is an aerosol formulation or a non-aerosol formulation, and wherein the formulation is selected from the group consisting of a gel, a mousse, a spray, a serum and a wax.

8. A process for restyling human hair comprising
applying to the human hair a composition according to any of claims 1 to 7,
thereafter heat-treating the hair, together with the composition on its surface, at a temperature of 145 to 250° C, preferably 150 to 230° C, more preferably 150 to 190°C, more preferably 160 to 185° C, wherein the hair-style is changed during this heat treatment,
thereafter allowing the hair to cool down to a temperature of below 100° C,
and thereafter changing the hair style by heat-treating the hair, together with the composition on its surface, at a temperature of 145 to 250° C, preferably 150 to 230°C, more preferably 150 to 190°C, more preferably 160 to 185° C.

9. The process according to claim 8, wherein the changing of the hair style is carried out with a styling device selected from the group consisting of a straightening iron, curling iron or a crimper.

10. The use of a composition as defined in any of claims 1 to 7 for restyling human hair, wherein the restyling comprises
applying to the human hair the polymer or the composition,
thereafter heat-treating the hair, together with the polymer or with the composition on its surface, at a temperature of 145 to 250° C, preferably 150 to 230° C, more preferably 150 to 190°C, more preferably 160 to 185° C, wherein the hair-style is changed during this heat treatment,
thereafter allowing the hair to cool down to a temperature of below 100° C,
and thereafter changing the hair style by heat-treating the hair, together with the polymer or with the composition on its surface, at a temperature of 145 to 250° C, preferably 150 to 230° C, more preferably 150 to 190°C, more preferably 160 to 185°C.

## Patentansprüche

1. Zusammensetzung, umfassend
ein Polymer, wobei dieses Polymer wasserlöslich ist in einen Schmelzpunkt von mindestens 145°C, vorzugsweise mindestens 160°C, aufweist und Sulfonsäuregruppen umfasst,
und ein organisches Amin mit mindestens zwei Aminogruppen, wobei das organische Amin mit mindestens zwei Aminogruppen aus der Gruppe bestehend aus Ethylendiamin, Diethylentriamin, Triethylentetramin, Tetraethylenpentamin und Tetrahydroxypropylethylendiamin ausgewählt ist,
und gegebenenfalls ferner kosmetische Inhaltsstoffe, die vorzugsweise aus der Gruppe bestehend aus Lösungsmitteln, Rheologiemodifikatoren, Konservierungsstoffen, Tensiden, Emulgatoren, Parfümen, Pigmenten und Farbmitteln ausgewählt sind.

2. Zusammensetzung nach Anspruch 1, wobei das Polymer aus der Gruppe bestehend aus Polyacrylamidomethylpropansulfonsäure und Polystyrolsulfonsäure ausgewählt ist, wobei es sich bei dem Polymer vorzugsweise um Polyacrylamidomethylpropansulfonsäure handelt.

3. Zusammensetzung nach Anspruch 1 oder 2, wobei es sich bei dem organischen Amin mit mindestens zwei Aminogruppen um Tetrahydroxypropylethylendiamin handelt.

4. Zusammensetzung nach einem der Ansprüche 1 bis 3, wobei das Polymer in einer Menge von 0,1 bis 10 Gew.-%, Vorzugsweise 0,5 bis 2,5 Gew.-%, vorliegt.

5. Zusammensetzung nach einem der Ansprüche 1 bis 4, wobei das organische Amin mit mindestens zwei Aminogruppen in einer Menge vorliegt, die zu teilweiser oder vollständiger Neutralisation des Polymers führt, und wobei die Zusammensetzung einen pH-Wert von 1 bis 14, vorzugsweise von 3 bis 9, aufweist.

6. Zusammensetzung nach einem der Ansprüche 1 bis 5, wobei die Zusammensetzung mindestens ein weiteres Polymer umfasst, wobei dieses weitere Polymer vorzugsweise in einer Konzentration von 0,1 bis 10 Gew.-% vorliegt und aus der Gruppe bestehend aus nichtionischen, anionischen oder kationischen Polymeren ausgewählt ist.

7. Zusammensetzung nach einem der Ansprüche 1 bis 6, wobei es sich bei der Zusammensetzung um eine Aerosolformulierung oder eine Nicht-Aerosolformulierung handelt und wobei die Formulierung aus der Gruppe bestehend aus einem Gel, einer Mousse, einem Spray, einem Serum und einem Wachs ausgewählt ist.

8. Verfahren zum Umfrisieren von menschlichem Haar, das Folgendes umfasst:
Aufbringen einer Zusammensetzung nach einem der Ansprüche 1 bis 7 auf das menschliche Haar,
danach Wärmebehandeln des Haars zusammen mit der Zusammensetzung auf seiner Oberfläche bei einer Temperatur von 145 bis 250 °C, vorzugsweise 150 bis 230 °C, weiter bevorzugt 150 bis 190 °C, weiter bevorzugt 160 bis 185 °C, wobei die Frisur während dieser Wärmebehandlung verändert wird,
danach Abkühlenlassen des Haars auf eine Temperatur unter 100 °C
und danach Verändern der Frisur durch Wärmebehandeln des Haars zusammen mit der Zusammensetzung auf seiner Oberfläche bei einer Temperatur von 145 bis 250 °C, vorzugsweise 150 bis 230 °C, weiter bevorzugt 150 bis 190 °C, weiter bevorzugt 160 bis 185 °C.

9. Verfahren nach Anspruch 8, wobei das Verändern der Frisur mit einer aus der Gruppe bestehend aus einem Glätteisen, einem Lockenstab oder einem Kreppeisen ausgewählten Frisiervorrichtung durchgeführt wird.

10. Verwendung einer Zusammensetzung gemäß einem der Ansprüche 1 bis 7 zum Umfrisieren von menschlichem Haar, wobei das Umfrisieren Folgendes umfasst:
Aufbringen des Polymers bzw. der Zusammensetzung auf das menschliche Haar,
danach Wärmebehandeln des Haars zusammen mit dem Polymer bzw. mit der Zusammensetzung auf seiner Oberfläche bei einer Temperatur von 145 bis 250 °C, vorzugsweise 150 bis 230 °C, weiter bevorzugt 150 bis 190 °C, weiter bevorzugt 160 bis 185 °C, wobei die Frisur während dieser Wärmebehandlung verändert wird,
danach Abkühlenlassen des Haars auf eine Temperatur unter 100 °C
und danach Verändern der Frisur durch Wärmebehandeln des Haars zusammen mit dem Polymer bzw. mit der Zusammensetzung auf seiner Oberfläche bei einer Temperatur von 145 bis 250 °C, vorzugsweise 150 bis 230 °C, weiter bevorzugt 150 bis 190°C, weiter bevorzugt 160 bis 185°C.

## Revendications

1. Composition comprenant
un polymère, ce polymère étant soluble dans l'eau et possédant un point de fusion d'au moins 145 °C, préférablement d'au moins 160 °C, et comprenant des groupes de type acide sulfonique,
et une amine organique possédant au moins deux groupes amino, l'amine organique possédant au moins deux groupes amino étant choisie dans le groupe constitué par l'éthylènediamine, la diéthylènetriamine, la triéthylènediamine, la tétraéthylènepentamine et la tétrahydroxypropyl-éthylènediamine,
et éventuellement des ingrédients cosmétiques supplémentaires, préférablement choisis dans le groupe constitué par des solvants, des modificateurs de rhéologie, des conservateurs, des tensioactifs, des émulsifiants, des parfums, des pigments et des agents colorants.

2. Composition selon la revendication 1, le polymère étant choisi dans le groupe constitué par un poly(acide acrylamidométhylpropanesulfonique) et un poly(acide styrènesulfonique), préférablement le polymère étant un poly(acide acrylamidométhylpropanesulfonique).

3. Composition selon la revendication 1 ou 2, l'amine organique possédant au moins deux groupes amino étant la tétrahydroxypropyl-éthylènediamine.

4. Composition selon l'une quelconque des revendications 1 à 3, le polymère étant présent en une quantité allant de 0,1 à 10 % en poids, préférablement de 0,5 à 2,5 % en poids.

5. Composition selon l'une quelconque des revendications 1 à 4, l'aminé organique possédant au moins deux groupes amino étant présente en une quantité qui conduit à une neutralisation partielle ou complète du polymère, et la composition possédant une valeur de pH de 1 à 14, préférablement de 3 à 9.

6. Composition selon l'une quelconque des revendications 1 à 5, la composition comprenant au moins un polymère supplémentaire, ce polymère supplémentaire étant préférablement présent à raison d'une concentration de 0,1 à 10 % en poids et étant choisi dans le groupe constitué par des polymères non ioniques, anioniques ou cationiques.

7. Composition selon l'une quelconque des revendications 1 à 6, la composition étant une formulation d'aérosol ou une formulation non de type aérosol, et la formulation étant choisie dans le groupe constitué par un gel, une mousse, un spray, un sérum et une cire.

8. Procédé pour le relooking de cheveux humains comprenant
l'application aux cheveux humains d'une composition selon l'une quelconque des revendications 1 à 7, ensuite, le traitement thermique des cheveux, conjointement avec la composition sur sa surface, à une température de 145 à 250 °C, préférablement de 150 à 230 °C, plus préférablement de 150 à 190 °C, plus préférablement de 160 à 185 °C, le style capillaire étant modifié pendant ce traitement thermique,
ensuite, le fait de laisser les cheveux refroidir jusqu'à une température inférieure à 100 °C,
et ensuite la modification du style capillaire par traitement thermique des cheveux, conjointement avec la composition sur sa surface, à une température de 145 à 250 °C, préférablement de 150 à 230 °C, plus préférablement de 150 à 190 °C, plus préférablement de 160 à 185 °C.

9. Procédé selon la revendication 8, la modification du style capillaire étant mise en œuvre avec un dispositif de coiffage choisi dans le groupe constitué par un fer à lisser, un fer à friser ou un fer à gaufrer.

10. Utilisation d'une composition telle que définie dans l'une quelconque des revendications 1 à 7 pour le relooking de cheveux humains, le relooking comprenant
l'application aux cheveux humains du polymère ou de la composition,
ensuite, le traitement thermique des cheveux, conjointement avec le polymère ou avec la composition sur sa surface, à une température de 145 à 250 °C, préférablement de 150 à 230 °C, plus préférablement de 150 à 190 °C, plus préférablement de 160 à 185 °C, le style capillaire étant modifié pendant ce traitement thermique,
ensuite, le fait de laisser les cheveux refroidir jusqu'à une température inférieure à 100 °C,
et ensuite la modification du style capillaire par traitement thermique des cheveux, conjointement avec le polymère ou avec la composition sur sa surface, à une température de 145 à 250°C, préférablement de 150 à 230 °C, plus préférablement de 150 à 190 °C, plus préférablement de 160 à 185°C.
